# EUROPEAN PATENT SPECIFICATION

(11) **EP 1 432 879 B1**
(45) Date of publication and mention of the grant of the patent: **01.02.2006**
(21) Application number: 02760434.7
(22) Date of filing: 26.09.2002
(51) Int. Cl.: E03D 9/00, A61L 9/05

(54) **A DISPENSER**
EIN SPENDER
DISTRIBUTEUR

(30) Priority: 27.09.2001 GB 0123250
(43) Date of publication of application: 30.06.2004
(73) Proprietor: Robert McBride Ltd, Middleton, Manchester M24 4DP (GB)
(72) Inventor: SMITH, Nigel Peter, Lantern Cottage, Devon TQ12 6HH (GB)
(74) Representative: Newstead, Michael John
(86) International application number: PCT/GB2002/004371
(87) International publication number: WO 2003/027405

(56) References cited:
- EP-A- 1 203 844
- WO-A-92/20876
- DE-A- 3 611 265
- DE-C- 72 266

## Description

### Field of the Invention

This invention relates to a dispenser and, in particular, to a dispenser adapted for suspension from the rim of a toilet bowl to dispense one or more active substances, such as odour neutralising and/or air freshening preparations, into the toilet bowl; or into the near vicinity of the toilet bowl.

### Background to the Invention

Devices suspended from the rims of toilet bowls, to dispense freshening and/or cleaning preparations, are well known and are commonly referred to as rimstick devices. In one form, a rimstick device comprises a cage used to retain a replaceable block impregnated with disinfectant and/or freshening agent. When the toilet is flushed, the flush water passes through the cage and degrades part of the block. The active substances from the block are entrained in the water and thus pass out into the toilet bowl.

More recently rimstick devices have become available which include a porous pad in communication with a reservoir of a viscous liquid cleaning and freshening substance. The liquid substance saturates the pad and is drawn out when flush water is directed over the pad. As active cleaning liquid is flushed from the pad, further liquid is supplied from the reservoir to re-saturate the pad. An example of this type of device is described and claimed in European Patent Application 0 785 315.

Quite apart from the above, it is also common practice to place an air freshening and/or odour neutralising device in a room containing a toilet. This device may take a number of forms, one particularly common form being a plastics moulding containing a gel impregnated with an air treatment substance. Upon exposure to air, the gel volatizes and the air treatment substance comes off in the form of vapours. These vapours pass through a perforated surface of the plastics moulding and into the volume of the room containing the toilet. Typically an air freshening device of the type just described is placed on a window sill or in some other location within the toilet room where it is likely the device will be subjected to an air-stream passing over the surface thereof. Such an air-stream encourages the vapours to emanate from the gel and it will also be appreciated that the air-stream also distributes the vapours throughout the volume of the toilet room.

It is an object of this invention to provide a simple yet effective form of dispenser which combines, in a novel and inventive manner, aspects of the rimstick and air-freshening devices described above.

EP-A-1 203 844 and WO-A-92 20 876 each discloses a dispenser having the precharacterising features of claim 1.

### Summary of the Invention

According to the present invention from one aspect, there is provided a dispenser for an air modifying agent, said dispenser including:
a closed housing containing an air modifying substance; and
location means constructed and arranged to suspend said housing from the rim of a toilet bowl when in use,
said dispenser being characterized in that part of said housing is defined by or includes a vapour-permeable membrane, said membrane being configured to permit the egress of air modifying vapours from said housing whilst preventing the passage of liquids out of, and into, said housing.

Preferably the construction and arrangement of said housing is such that, when said housing is suspended from the rim of a toilet bowl, said membrane is located in or near to the water path when said toilet is flushed.

Preferably said air modifying substance comprises a perfume and/or an odour neutraliser.

Preferably said housing is defined, principally, by a plastics moulding, said location means comprising an integral hook to facilitate suspension of said housing from said rim.

Alternatively said location means may comprise a carrier provided with a hook, said housing being adapted for engagement with said carrier.

According to the present invention from another aspect, there is provided a method for dispensing, in the vicinity of a toilet, vapours from an air modifying substance, said method including:
locating an air modifying substance in a closed housing; and
attaching said housing to the rim of a toilet bowl so that air modifying vapours emanating from said air modifying substance can pass from said housing into the vicinity of said toilet bowl,
said method being characterised in that a vapour-permeable membrane is employed to define at least part of said housing, said membrane being configured to permit only air modifying vapours to pass therethrough whilst preventing liquids passing out of, and into, said housing.

Preferably said method further includes locating said housing in relation to said rim so that said membrane lies substantially in the flush water path of said toilet.

Many variations in the way the invention may be performed will present themselves to those skilled in the art upon reading the following description. The description which follows should not be regarded as limiting but rather, as an illustration only of one mode of performing the invention as defined in the claims. Where possible, a description of any element or component should be taken as including any or all equivalents thereof whether or not specifically mentioned. The scope of the invention is determined solely by the appended claims.

### Brief Description of the Drawings

One form of dispenser embodying the various aspects of the invention will now be described with reference to the accompanying drawings in which:
- Figure 1:: shows a front elevation of a first form of dispenser according to the invention;
- Figure 2:: shows a view along the line II-II in Figure 1;
- Figure 3:: shows a front elevation of a second form of dispenser according to the invention; and
- Figure 4:: shows a view along the line IV-IV in Figure 3.

### Detailed Description of Working Embodiment

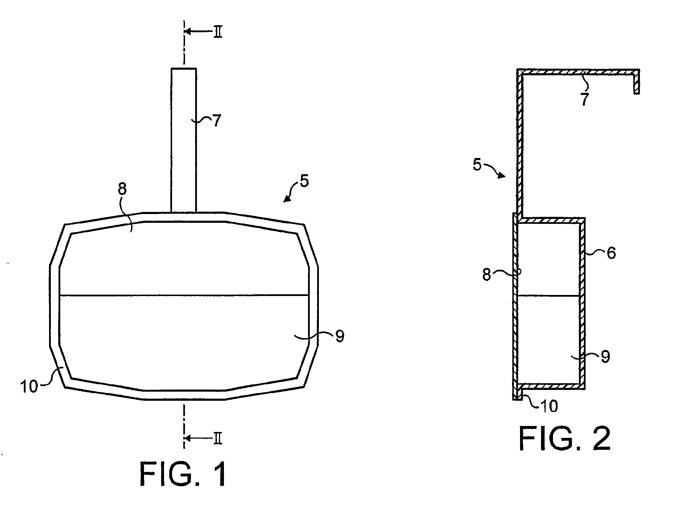

Referring firstly to Figures 1 and 2, a first form of dispenser 5 is shown for dispensing an air modifying agent into the vicinity of a toilet bowl (not shown). As can be seen the dispenser includes a housing 6 having a hook 7 to allow the housing to be suspended for the rim of a toilet bowl. Part of the housing wall includes, or is defined by, a semi-permeable membrane 8.

Located within the housing is an air modifying substance 9 of the type which volatizes and gives off air modifying vapours. The form of the air modifying substance does not constitute part of the invention but may, for example, comprise a gel or a liquid. As the substance 9 volatizes, the vapours pass through the membrane 8 and into the toilet bowl or the region surrounding the toilet bowl.

Particularly satisfactory results have been obtained in testing using a liquid fragrance, either on its own or with a small (around 2%) amount of thickener to increase viscosity.

The air modifying agent is preferably an air freshening and/or odour neutralising agent.

In the form shown in Figures 1 and 2, the housing 6 is a simple open-sided moulded plastics tray with the hook 7 being an integral part of that moulding. The membrane may be of any suitable form and may be adhered or otherwise fixed to flange 10 which surrounds the open face of the tray. It will be noted that the relationship of the tray and hook is such that the membrane faces outward when the hook is placed over the rim of a toilet bowl. In this way, the surface of the membrane is exposed to flush water during use of the toilet, and this creates an airflow over the membrane which aids the extraction and dispersion of air modifying vapours.

The membrane may be of any form known in the art and may, for example, comprise a polyethylene sheet of 50 to 120 microns thickness. The only essential requirement is that the membrane allows vapours to escape yet does not allow the ingress of liquids. The thickness and composition of the membrane can be varied to achieve the desired rate of vapour dispersion through the membrane, thus establishing the life of the product. Typically the membrane will be a polyethylene extrusion but may also be a co-extrusion of a number of materials. Prior to use (during storage) an impermeable sealing foil (not shown) may be adhered over the membrane 8 to prevent the egress of air modifying vapours.

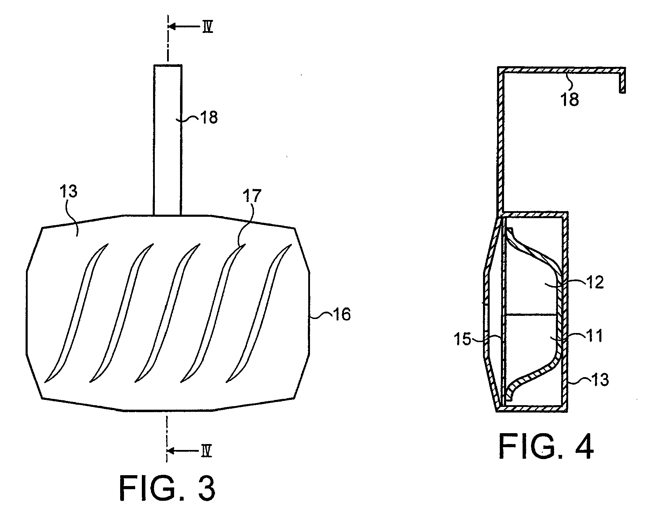

One alternative form of device is shown in Figures 3 and 4. In this device, the air modifying substance 11 is contained in a separate refill cartridge 12 which is slipped into a more permanent form of carrier 13. The cartridge 12 comprises a simple plastics tray 14 with membrane 15 covering the open face thereof. Upon the air modifying substance in the cartridge 12 becoming depleted, the tray 14 is extracted and discarded, and a replacement cartridge inserted into carrier 13.

The cartridge 12 is preferably inserted into carrier 13 through open end 16 and the carrier may include grills or openings 17 to assist in allowing the air modifying vapours to disperse into the vicinity of the toilet bowl.

Integrally formed with the carrier is a suspension hook 18 which functions in the known manner.

It will thus be appreciated that the present invention, particularly in the case of the two preferred devices described above, provides a simple yet effective form of rim mounted dispenser for dispensing air freshening and/or odour neutralising substances directly adjacent the site at which such substances need to act.

## Claims

1. A dispenser (5) for an air modifying agent, said dispenser (5) including:
a closed housing (6, 12) containing an air modifying substance (9, 11); and
location means (7, 18) constructed and arranged to suspend said housing (6, 12), from the rim of a toilet bowl when in use,
said dispenser (5) being **characterized in that** part of said housing (6, 12) is defined by or includes a vapour-permeable membrane (8, 15), said membrane (8, 15) being configured to permit the egress of air modifying vapours from said housing (6, 12) whilst preventing the passage of liquids out of, and into, said housing (6, 12).

2. A dispenser as claimed in claim 1 wherein said housing (6, 12) is constructed and arranged for location beneath said rim when in use.

3. A dispenser as claimed in claim 1 or claim 2 wherein the construction and arrangement of said housing (6, 12) is such that, when said housing is suspended from the rim of a toilet bowl, said membrane (8, 15) is located in or near to the water path when said toilet is flushed.

4. A dispenser as claimed in any one of claims 1 to 3 wherein said air modifying substance (9, 11) comprises a perfume and/or an odour neutraliser.

5. A dispenser as claimed in any one of the preceding claims wherein said housing (6, 12) is defined, principally, by a plastics moulding, said location means (7, 18) comprising an integral hook to facilitate suspension of said housing from said rim.

6. A dispenser as claimed in any one of claims 1 to 4 wherein said location means (18) comprises a carrier (13) provided with a hook, said housing (12) being adapted for engagement with said carrier (13).

7. A method for dispensing, in the vicinity of a toilet, vapours from an air modifying substance (9, 11), said method including:
locating an air modifying substance (9, 11) in a closed housing (6, 12); and
attaching said housing (6, 12) to the rim of a toilet bowl so that air modifying vapours emanating from said air modifying substance can pass from said housing (6, 12) into the vicinity of said toilet bowl,
said method being **characterized in that** an vapour-permeable membrane (8, 15) is employed to define at least part of said housing (6, 12), said membrane being configured to permit only air modifying vapours to pass there-through whilst preventing liquids passing out of, and into, said housing (6, 12).

8. A method as claimed in claim 7 further including locating said housing (6, 12) in relation to said rim so that said membrane (8,15) lies substantially in the flush water path of said toilet.

## Patentansprüche

1. Spender (5) für ein luftveränderndes Mittel mit:
einem geschlossenen Gehäuse (6, 12), das eine luftverändernde Substanz (9, 11) enthält; und
einer Anordnungsvorrichtung (7, 18), die konstruiert und ausgebildet ist, das Gehäuse (6, 12) an dem Rand einer Toilettenschüssel bei Verwendung aufzuhängen, **dadurch gekennzeichnet, dass** ein Teil des Gehäuses (6, 12) durch eine dampfdurchlässige Membrane (8, 15) definiert ist oder diese aufweist, die konfiguriert ist, den Austritt luftverändernder Dämpfe aus dem Gehäuse (6, 12) zu erlauben, während sie hingegen den Durchgang von Flüssigkeiten aus und in das Gehäuse (6, 12) verhindert.

2. Spender nach Anspruch 1, bei dem das Gehäuse (6, 12) zur Anordnung unter dem Rand bei Verwendung konstruiert und ausgebildet ist.

3. Spender nach Anspruch 1 oder 2, bei dem die Konstruktion und Ausbildung des Gehäuses (6, 12) derart ist, dass, wenn das Gehäuse an dem Rand einer Toilettenschüssel aufgehängt ist, die Membrane (8, 15) in oder in der Nähe des Wasserweges angeordnet ist, wenn die Toilette gespült wird.

4. Spender nach einem der Ansprüche 1 bis 3, bei dem die luftverändernde Substanz (9, 11) ein Parfüm oder einen Geruchsneutralisierer aufweist.

5. Spender nach einem der vorstehenden Ansprüche, bei dem das Gehäuse (6, 12) im Prinzip durch eine Kunststoffform definiert ist und bei dem die Anordnungsvorrichtung (7, 18) einen einstückigen Haken aufweist, um die Aufhängung des Gehäuses an dem Rand zu erleichtern.

6. Spender nach einem der Ansprüche 1 bis 4, bei dem die Anordnungsvorrichtung (18) einen mit einem Haken versehenen Tragrahmen (13) aufweist und das Gehäuse (12) zum Eingriff mit dem Tragrahmen (13) angepasst ist.

7. Verfahren zum Spenden von Dämpfen aus einer luftverändernden Substanz (9, 11) in der Nähe einer Toilette mit:
Anordnen einer luftverändernden Substanz (9, 11) in einem geschlossenen Gehäuse (6, 12); und
Anbringen des Gehäuses (6, 12) an dem Rand einer Toilettenschüssel, so dass luftverändernde Dämpfe, die aus der luftverändernden Substanz ausströmen, aus dem Gehäuse (6, 12) in die Nähe der Toilettenschüssel gelangen können, **dadurch gekennzeichnet, dass** eine dampfdurchlässige Membrane (8, 15) verwendet wird, um zumindest einen Teil des Gehäuses (6, 12) zu definieren, wobei die Membrane konfiguriert ist, nur luftverändernde Dämpfe zu erlauben, hindurch zu gelangen, während Flüssigkeiten gehindert werden, aus und in das Gehäuse (6, 12) zu gelangen.

8. Verfahren nach Anspruch 7, das ferner Anordnen des Gehäuses (6, 12) in Bezug auf den Rand derart aufweist, dass die Membrane (8, 15) im Wesentlichen in dem Spülwasserweg der Toilette liegt.

## Revendications

1. Distributeur (5) d'un agent de modification de l'air, le distributeur (5) comprenant :
un boîtier (6, 12) fermé contenant une substance (9, 11) de modification de l'air ; et
des moyens (7, 18) de localisation agencés et conçus pour suspendre le boîtier (6, 12) au rebord d'une cuvette de W.-C. lorsqu'il est utilisé,
le distributeur (5) étant **caractérisé en ce qu'**une partie du boîtier (6, 12) est définie par une membrane (8, 15) perméable à la vapeur ou inclut une membrane de ce genre, la membrane (8, 15) étant configurée pour permettre la sortie de vapeurs de modification de l'air du boîtier (6, 12) tout en empêchant le passage de liquides sortant du boîtier (6, 12) et y entrant.

2. Distributeur suivant la revendication 1, dans lequel le boîtier (6, 12) est agencé et conçu pour être placé en dessous du rebord lorsqu'il est utilisé.

3. Distributeur suivant la revendication 1 ou la revendication 2, dans lequel l'agencement et la conception du boîtier (6, 12) sont tels que lorsque le boîtier est suspendu au rebord de la cuvette du W.-C., la membrane (8, 15) est placée dans le trajet de l'eau ou à proximité du trajet de l'eau lorsque la chasse d'eau est tirée.

4. Distributeur suivant l'une quelconque des revendications 1 à 3, dans lequel la substance (9, 11) de modification de l'air comprend un parfum et/ou un agent de neutralisation des odeurs.

5. Distributeur suivant l'une quelconque des revendications précédentes, dans lequel le boîtier (6, 12) est défini principalement par une pièce moulée en matière plastique, les moyens (7, 18) de localisation comprenant un crochet d'un seul tenant pour faciliter la suspension du boîtier au rebord.

6. Distributeur suivant l'une quelconque des revendications 1 à 4, dans lequel les moyens (18) de localisation comprennent un support (13) muni d'un crochet, le boîtier (12) étant conçu pour coopérer avec le support (13).

7. Procédé de distribution, à proximité d'un W.-C., de vapeurs d'une substance (9, 11) de modification de l'air, le procédé comprenant :
la mise d'une substance (9, 11) de modification de l'air dans un boîtier (6, 12) fermé ; et
la fixation du boîtier (6, 12) au rebord d'une cuvette des W.-C., de sorte que des vapeurs de modification de l'air émanant de la substance de modification de l'air peuvent passer du boîtier (6, 12) à proximité de la cuvette des W.-C.,
le procédé étant **caractérisé en ce que** l'on emploie une membrane (8, 15) perméable à la vapeur pour définir au moins une partie du boîtier (6, 12), la membrane étant configurée pour permettre seulement à des vapeurs de modification de l'air d'y passer à travers, alors que des liquides sont empêchés de sortir du boîtier (6, 12) et d'y entrer.

8. Procédé suivant la revendication 7, comprenant en outre la mise en place du boîtier (6, 12) par rapport au rebord, de façon à ce que la membrane (8, 15) se trouve sensiblement dans le trajet de l'eau de chasse du W.-C.
